# EUROPEAN PATENT SPECIFICATION

(11) **EP 3 880 793 B1**
(45) Date of publication and mention of the grant of the patent: **25.03.2026**
(21) Application number: 19801025.8
(22) Date of filing: 12.11.2019
(51) Int. Cl.: C12M 3/00, C12M 1/12, C12M 1/33

(54) **HANDLING OF MEMBRANE-SHAPED TISSUE**
HANDHABUNG VON MEMBRANFÖRMIGEM GEWEBE
MANIPULATION DE TISSUS EN FORME DE MEMBRANE

(30) Priority: 12.11.2018 EP 18205799
(43) Date of publication of application: 22.09.2021
(73) Proprietor: Universiteit Antwerpen, 2000 Antwerpen (BE)
(72) Inventor: CHAKKAR, Yassin, 3000 Leuven (BE); GOETHIJN, Frank, 9000 Gent (BE); ZAKARIA, Nadia, Somerville MA 02144 (US)
(74) Representative: Winger
(86) International application number: PCT/EP2019/080972
(87) International publication number: WO 2020/099373

(56) References cited:
- WO-A1-2007/080600
- WO-A1-2012/045368
- DE-A1- 102013 113 146
- US-A- 6 048 723
- US-A1- 2008 076 170
- US-A1- 2017 037 353

## Description

### Field of the invention

The invention relates to the field of tissue culture, cell therapy and regenerative medicine. More specifically it relates to a tool for fixating and handling membrane-shaped tissue and a corresponding method for fixating and handling the membrane-shaped tissue.

### Background of the invention

Tissue culture is the process of taking cells from a human, an animal or a plant and placing them in a controlled artificial environment to encourage growth and multiplication. They can be derived from the tissue directly, using enzymatic or mechanical methods, or they can be taken from a cell line or cell strain. The source of these cells can vary a lot, for example they can be harvested from the eye, skin, bone, kidney,... Such cell cultures typically are grown on membrane-shaped tissue, for which often Amnion tissue is used.

Experiments with membrane-shaped tissue require specific mechanical handling like fixation, stretching, immobilizing, inserting in multiwell plates, storing, observation, recollecting, cutting or other manipulations. Besides the mechanical manipulations these experiments also include chemical treatments like sterilizing, adding and removing substances...

An important step in the handling of membrane-shaped tissue is the spanning of the tissue, which is currently being done between two rings. The typical process of spanning currently includes spreading the membrane-shaped tissue, place a first ring on the membrane, fold the sides of the membrane-shaped tissue over the ring using tweezers, lift the ring with the membrane-shaped tissue and position it in or over a second ring, tightly press the two rings on each other and thereafter further handle the membrane-shaped tissue.

DE 10 2013 113146 A1 discloses two frames for fixing a biocompatible material, both frames to be connected to span a culture plate between them wherein a ring is spanned over the first frame to fix the membrane. US 2008/076170 A1 discloses a cell culture insert and a locking ring adapted to be fitted around the body of the cell culture insert and a cell culture membrane to be fixed between the ring and the cell culture body.

In general it takes quite long per membrane to go through all the steps of fixating a membrane. Furthermore, a fair amount of fails when spanning the membrane can be noticed, which appear to depend on the size of the membrane. Especially when the membrane is small, failure may occur because the researcher typically attempts to obtain the biggest spanned surface for the membrane. A bigger surface results in better cell growth and better results in general. Often when the tissue comes of the ring, the tissue will completely cramp up again, requiring to spread it again, requiring significant time.

The current technology to fixate membranes therefore does not fulfil the needs of the users.

### Summary of the invention

It is an object of embodiments of the present invention to provide a good and efficient system and device for handling membrane-shaped tissues, e.g. for assisting studies (research and clinical) in cell insert cultures. It is an advantage of embodiments of the present invention that a simple pushing-tool is provided allowing user-friendly and efficient handling of membrane-shaped tissues.

It is an advantage of embodiments of the present invention that de-attachment of the membrane shaped tissue from the membrane spanning device, when moving the membrane shaped tissue, for example from the preparation environment towards a well plate, can be avoided.

It is an advantage of embodiments of the present invention that the spanning device can be easily handled.

It is an advantage of embodiments of the present invention that a membrane-shaped tissue can be fixated with equal tension to all sides of the tissue.

It is an advantage of embodiments of the present invention that easy inspection of the membrane shaped tissue as well as of cell cultures installed on the membrane shaped tissue can be performed.

It is an advantage of embodiments of the present invention that, when a cutting support is used, the cutting support can be easily replaced. The cutting support may comprise a grip for easily removing the cutting support from the platform. The cutting support may for example be made of stainless steel, allowing punching of the membrane so that it is cut. It is an advantage of embodiments of the present invention that cutting of the membrane can be performed by cutting against the edge of the cutting support surrounding the position of the ring.

It is an advantage of embodiments of the present invention that a system can be provided for fixating a membrane shaped tissue whereby the risk of puncture is low. It is to be noted that cells don't culture on a punctured membrane.

It is an advantage of embodiments of the present invention that the amount of useable membrane shaped tissue that is usable after fixation is optimized with respect to the total amount of surface area of tissue available.

It is an advantage of embodiments of the present invention that a system is provided allowing to use different sizes and therefore is compatible with different types of well-plates, e.g. fixation can be made for wells of a 6-well plate, a 12-well plate, or a 24-well plate.

It is an advantage of embodiments of the present invention that system is provided allowing to use different medium configurations, i.e. to work with a configuration wherein only a medium is present at the bottom side of the membrane, a configuration wherein different media is provided at both sides of the membrane or a configuration wherein the same media is provided at both sides of the membrane. The system thus provides flexibility on the amount and the position of where the medium is provided.

It is an advantage of embodiments according to the present invention that the system allows working with wet as well as dry membranes.

It is an advantage of embodiments of the present invention that inserting the spanning device with the spanned membrane shaped tissue into e.g. a well plate where typically research and/or further actions are performed, can be easily done.

It is an advantage of embodiments of the present invention that the spanning device fits the most common membranes, for example it fits membrane shaped tissue thicknesses between 20µm and 200µm.

It is an advantage of embodiments of the present invention that the systems can easily be used within a flow hood, since the movements that are required only are small movements.

The present invention relates to a tool, the use thereof and a method as set out in the appended claims.

It is an advantage of embodiments of the present invention that a user friendly system is provided for spanning a membrane shaped tissue. Furthermore, it is an advantage of embodiments of the present invention to provide a handling tool reducing the chance of damaging the membrane which typically is expensive and fragile tissue.

It is an advantage of embodiments of the present invention that the cutting support avoids that the base plate gets damage during cutting of the membrane-shaped tissue. It is an advantage of embodiments of the present invention that the cutting support can easily be replaced, without need for replacing the full system.

It is an advantage of embodiments of the present invention that the membrane can be cut on the dish itself, thus resulting in less need for manipulation and a more user-friendly handling of membrane shaped tissues, e.g. during cell insert culture studies. It is an advantage of embodiments of the present invention that the membrane can be cut in such a way that little loss of tissue occurs.

It is an advantage of embodiments of the present invention that the combination of different diameters in one device allows enabling efficient use of donor tissue, as it allows optimizing the amount of spanned surface of the tissue. It is an advantage of embodiments of the present invention that the tools allow for fitting different sized wells of well plates.

Particular and preferred aspects of the invention are set out in the accompanying independent and dependent claims.

These and other aspects of the invention will be apparent from and elucidated with reference to the embodiment(s) described hereinafter.

The handling means may comprise a round shaped end portion for holding the flexible element, wherein the round shaped end portion comprises slots for providing a resilient action when holding the flexible element.

The handling means may comprise holding arms with a round shaped end portion for holding the flexible element.

The holding arms may be arranged for providing a clamping function.

The round shaped end portion may comprise slots for providing a resilient action when holding the flexible element.

The holding arms may be arranged so as to allow a clamping function with the round shaped end portion for clamping the flexible element.

The round shaped end portion may be shaped for providing two, three or more contact points or contact areas at different positions along the perimeter of the flexible element, when the flexible element is positioned in the handling means.

The present invention also relates to a method for fixating and handling membrane-shaped tissue for research or clinical use, the method comprising
positioning at least one round shaped spanning element on a platform using a positioning element determining a position on the platform,
spreading the membrane-shaped tissue over the round shaped spanning element,
positioning at least one flexible element over the round shaped spanning element for spanning the membrane shaped tissue using a handling means, and
removing the combination of the round shaped spanning element and the flexible element when the membrane is spanned from the platform.

The present invention also relates to the use of a tool as described above for spanning of an amniotic membrane. Particular and preferred aspects of the invention are set out in the accompanying independent and dependent claims.

These and other aspects of the invention will be apparent from and elucidated with reference to the embodiment(s) described hereinafter.

### Brief description of the drawings

FIG 1 illustrates and exploded view of a handling tool in accordance with embodiments of the present invention.
FIG 2 illustrates an exemplary base plate of a handling tool in accordance with embodiments of the present invention.
FIG 3 illustrates a detail of a platform and an inserted spanning element of a handling tool in accordance with embodiments of the present invention.
FIG 4 illustrates an exemplary flexible element of a handling tool in accordance with embodiments of the present invention.
FIG 5 illustrates an exemplary flexible element combined with a spanning element of a handling tool in accordance with embodiments of the present invention, for fixing a membrane.
FIG 6 illustrates an exemplary handling means holding a flexible element of a handling tool in accordance with embodiments of the present invention.
FIG 7 illustrates the bottom view of an exemplary handling means (with no flexible element) of a handling tool in accordance with embodiments of the present invention.
FIG 8 shows an exemplary workflow of a method of providing spanned membranes in accordance with embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a handling tool for providing membranes for cell cultures and/or membrane-shaped tissues, as well as spanning and handling said membranes and/or tissues, for example in research and/or clinical environments. The tool comprises a platform comprising a base plate having at least one positioning element for positioning a spanning element on the platform. It also comprises at least one round shaped spanning element adapted for being positioned on the platform using the positioning element. In some embodiments, the system is adapted or provided with different round shaped spanning elements, having different diameters or the same diameter. This allows to adjust the size of the spanned area, so that this can be selected as large as possible, or allows to span different membranes at the same time using the same platform. Typical diameters of the round shaped elements may for example be 11mm, 17,5mm and 30mm, measured from the outer diameter. It furthermore comprises at least one flexible element for positioning over the round shaped spanning element for spanning the membrane shaped tissue. The tool also comprises at least one handling means for handling the flexible element during positioning of the flexible element over the round shaped spanning element, and for handling the combination of the round shaped spanning element and the flexible element, when the membrane is spanned over the round shaped spanning element. The handling means may be configured for handling round shaped elements of a specific size and thus different handling means may be provided for differently sized round shaped spanning elements.

By way of illustration, embodiments of the present invention not being limited thereto, a particular example will be further described below.

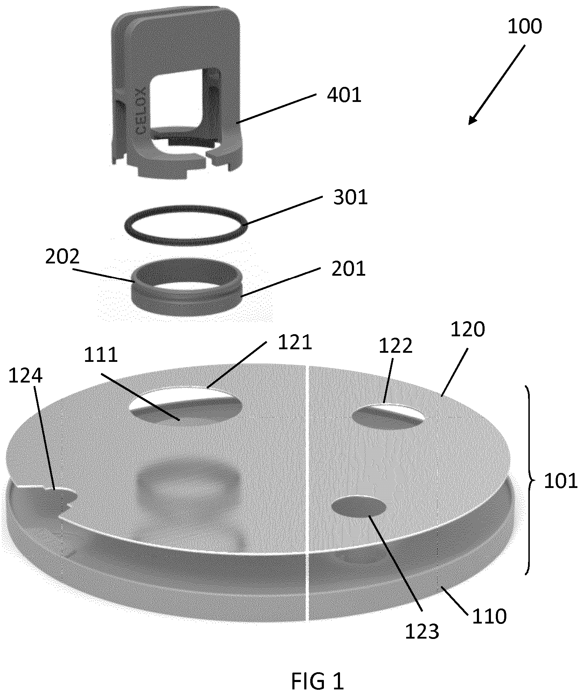

FIG 1 shows an exploded view of an exemplary handling tool 100 in accordance with some embodiments of the present invention. It includes a platform 101. The handling tool can be used as a support for manipulating (extending, cutting, etc.) a membrane, such as a tissue shaped as a membrane. In some embodiments of the present invention, the platform 101 comprises two pieces: a base plate 110 with one or more positioning elements 111, in the present example being protrusions, and a cutting support 120 with one or more holes 121, 122, 123. The one or more positioning elements on the base plate 110 can be inserted through the one or more holes of the cutting support. In some embodiments, when the cutting support 120 is positioned on the platform 101 (e.g. fitting the base plate 110), the upstanding protrusion of the base plate 110 extends through a corresponding hole 121 of the cutting support.

It further comprises at least one spanning element 201 with a ring shape (e.g. toroid), which can be positioned between on the positioning element 111 of the base plate 110 and the edge of a corresponding hole 121 of the cutting support 120, when the cutting support 120 is positioned on the platform.

It further comprises at least one flexible element 301, e.g. insert ring, which can be mounted to the spanning element, for spanning a membrane and fixing it to the spanning element.

The handling tool 100 further comprises a handling means 401, for example a clamp, which can be used for handling the insert ring, for example while positioning the flexible element over the spanning element. It can also handle the combination of the spanning element and insert ring while the membrane is spanned over the spanning element.

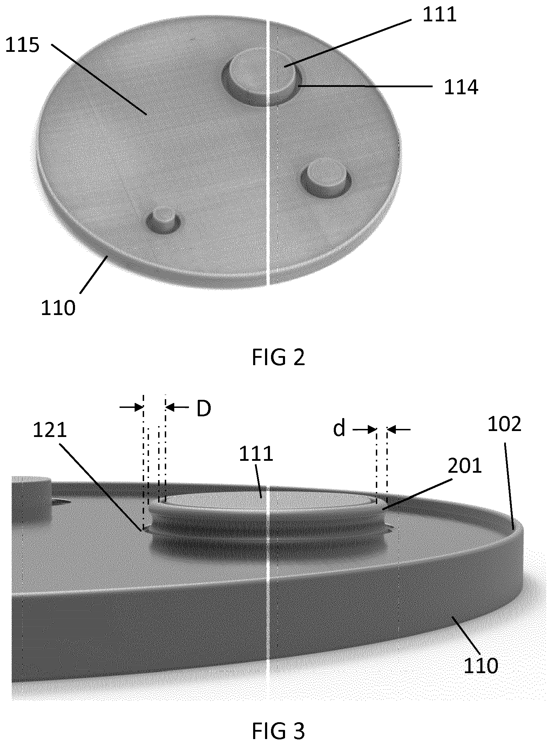

FIG 2 shows in detail an exemplary base plate 110 of embodiments of the present invention. It has three upstanding protrusions 111, 112, 113, although the present invention may comprise one, two or more than three positioning elements, e.g. protrusions. Multiple protrusions allow preparation of several spanned membranes in a single handling tool. The protrusion is cylindrical at least on its top part; it may be for example a substantially cylindrical upstanding protrusion 111. In some embodiments of the present invention, at least two of these substantially cylindrical protrusions 111, 112, 113, have different diameter, e.g. each of them has different diameter. This allows the preparation of spanned membranes and/or membrane-shaped tissues with different diameter, which may fit different sized wells of well plates, e.g. standard sized wells. The combination of different diameters allows efficient use of tissue, for example of donor tissue, which may be scarce, as it allows preparation of spanned membranes of different sizes for different purposes, cell growths, research or processing tools, etc. The upstanding protrusions may be considered to have different diameter in a direction parallel to the cutting support.

The present invention provides handling and spanning of membrane, and storage thereof, compatible with different types of well-plates, e.g. fixation can be made for wells of a 6-well plate, or a 12- well plate, even a 24- well plate.

In some embodiments of the present invention, the base plate 110 comprises materials compatible with biological and/or chemical products for handling of tissues and membranes. For example, it may be a plastics base plate, for instance polypropylene. The plastics base plate 110 may be produced by injection moulding. The present invention is not limited to these materials, and other materials can be used, not necessarily plastics. The material is advantageously chosen so that it can be sterilized at at least 80°C. It is to be noted that sterilization also could be performed at lower temperatures using H2O2 or Ethylene Oxide.

In some embodiments of the present invention, the base layer comprises blind cavities 114, or holes, from the bottom of which the handling means, e.g. upstanding protrusions 111, 112, 113 are provided. In some embodiments, the top surface of the upstanding protrusions (e.g. the top face of a cylindrical upstanding protrusion) protrudes out of the cavity, and in some embodiments it may extend through a circular hole of a cutting support. This may allow, for example, to fit different spanning devices of different heights.

Back to the exemplary embodiment of FIG 1 including a cutting support 120, the cutting support 120 includes at least one circular hole 121 which are configured so that the at least one upstanding protrusion 111 of the base plate 110 can extend through the hole 121. The cutting support may include further holes 122, 123 of different diameters at positions which allow inserting all of the upstanding protrusions of the base plate 110 on at least some of the holes 121, 122, 123.

The cutting support 120 may comprise hard materials, for allowing cutting the membrane. In some embodiments, it is hard enough so that the chance of damage or puncture is low. In some embodiments it also allows easy manipulation, spanning and slipping of the membrane. For example, the cutting support may comprise stainless steel. For example, the cutting support may be obtained by punching a stainless-steel plate. Alternatively, the cutting support may be made of a softer material, which is advantageous for the lifetime of the scalpel. A possible material that may be used can be self healing cutting mat PVC.

This allows cutting the membrane on the cutting support itself, thus resulting in less need for manipulation of the membrane. It provides a user-friendly handling of membrane shaped tissues, e.g. during cell insert culture studies. Because it provides a good cutting environment, the membrane can be cut in such a way that little loss of tissue occurs.

In some embodiments of the present invention the cutting support is replaceable, for example for cleaning, disposal or the like. The cutting support may comprise a grip 124 for easily removing the cutting support from the platform. The cutting support allow punching of the membrane so that it is cut through the holes. Additionally, or alternatively, cutting of the membrane can be performed easily by cutting against the edge of the cutting support (e.g. the edge of the hole 121) surrounding the position of the ring.

However, the present invention is not limited thereto. In some embodiments, cutting may be provided directly on the base plate, for which it is preferable that the surface 115 of the base plate 110 includes material suitable for cutting. In some embodiments, the top surface of the upstanding protrusions may be flush with the surface 115 of the base plate 110 where the cavities 114 are provided. This allows for a compact handling tool.

In some embodiments of the present invention, the platform has an upstanding edge 102 to allow medium to be provided in the platform, as shown in FIG 3. This can be used to hold the medium, and/or for influencing the environment during spanning and prior to transferring the membrane e.g. to a research or processing tool. For example, liquid mediums can be held. It can be used for avoidance of drying of the materials while preparing the membrane. The edge 102 is shown along the perimeter of the base plate 110 of FIG 3.

The spanning element (or spanning device) can hold the spanned membrane, for further manipulation. FIG 1 shows that the spanning element 201 may have a ring shape which can fit an upstanding protrusion 111. In some embodiments of the present invention, the spanning element may fit between the edge of a circular hole 121 of the cutting support 120 and the side of a protrusion 111 (e.g. the side wall of a cylindrical protrusion) of the base plate 110. For example, as shown in FIG 3, the spanning element may include an outer wall and an inner wall, and the distance d between at least a portion of the inner and the outer wall may be smaller than the distance D between the lateral wall of a protrusion 111 of the base plate 110 and the edge of its corresponding hole 121 of the cutting support 120, so the spanning element can at least partially fit between the protrusion and its circular hole.

Analogously, in some embodiments, it can also at least partially fit between the inner wall of the cavity 114 (shown in FIG 2) of the base plate 110 and the side wall of the upstanding protrusion 111.

In some embodiments of the present invention, the handling tool is configured so that the at least one round shaped spanning element sinks in the platform so that a top level is flush with the cutting support. This allows fixation of the membrane with similar or equal tension to all sides of the tissue. However, the present invention is not limited to this configuration, and in some embodiments, it may be flush with the top of the protrusion. In some embodiments, it may be flush with the base plate. In some embodiments, the top of the upstanding protrusion and the top of the spanning element may be flush with the cutting support or with the base layer.

The arrangement, in general, allows spanning easily a membrane over the spanning element and the upstanding protrusion.

The spanning element 201 and the flexible element 301 are adapted so they fit each other easily. For example, the spanning element and/or the flexible element may include a chamfer to ensure fitting. The flexible element may fit inside the spanning element, or it may fit outside. In some embodiments, the spanning element includes anchorage for a flexible flexible element 301. For example, FIG 1 shows that the outer wall of the spanning element 201 includes a groove 202, for example near one of its extremes. This allows proper grip of the flexible element 301 with little amount of excess membrane to provide fixation. However, other types of anchorage can be used, such as tabs, bumps and the like.

In some embodiments of the present invention, the spanning element 201 comprises materials compatible with biological and/or chemical products for handling of tissues and membranes, as discussed earlier regarding the base plate; it may include polypropylene, it may be produced by e.g. injection moulding, the present invention not being limited to these materials. The spanning element may include smooth surfaces to avoid tearing or puncturing the membrane, which may hinder the process of cell culture.

The flexible element 301 shown in FIG 1 is a flexible ring. The section of ring may be adapted for better insert to the spanning element 201. For example, the section of the ring 301 may be circular, allowing rolling of the ring 301 over the outer wall of the spanning element 201, thus allowing easy and fast insertion.

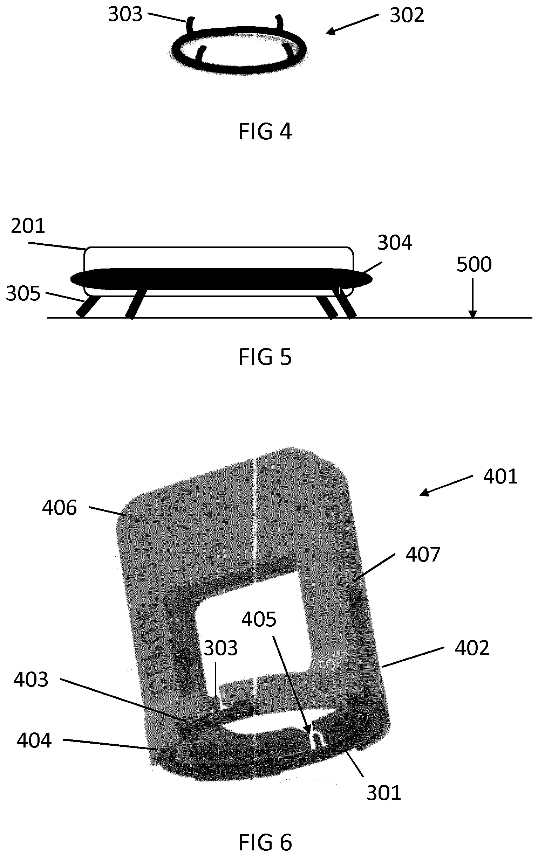

FIG 4 shows an exemplary flexible element 302, being an alternative from the flexible element 301 shown in FIG 1. The flexible element 302 comprises at least one protrusion or foot 303. Said foot can be used to handle the spanning element holding the membrane and can be lifted by the foot 301 of the ring, for example using tweezers. The feet may allow that liquid can be present below the tissue, e.g. once it is positioned in the well plate.

A plurality of feet may be provided, which can be shaped and positioned to reduce the chance of damaging the membrane when the spanned membrane, mounted on the spanning element 201, is transferred from the handling tool to e.g. a well. For example, FIG 5 shows that the flexible ring 304 with feet 305 maintains the spanning element 201, and any membrane attached thereto, lifted from a support surface 500, e.g. the bottom of a well. This avoids contact between any membrane or tissue provided and spanned on a bottom side of the spanning element 201 facing the support surface 500, and said support surface 500. In some embodiments (FIG 4) the feet 303 are bent inwards, which eases insertion in a well, as straight feet 305 (FIG 5) may touch the border of the well during insertion and manipulation of the spanned membrane.

The flexible element 301, 302 may be elastic, for example it may be a rubber, e.g. ethylene propylene diene monomer (EPDM) rubber.

Finally, the handling means 401 may include a clamp or the like, which may have the double function of inserting the flexible element 301 over the spanning element 201 and of grasping and handling the spanning element 201 with a membrane or membranous tissue attached thereto and fixed in place by the flexible element 301.

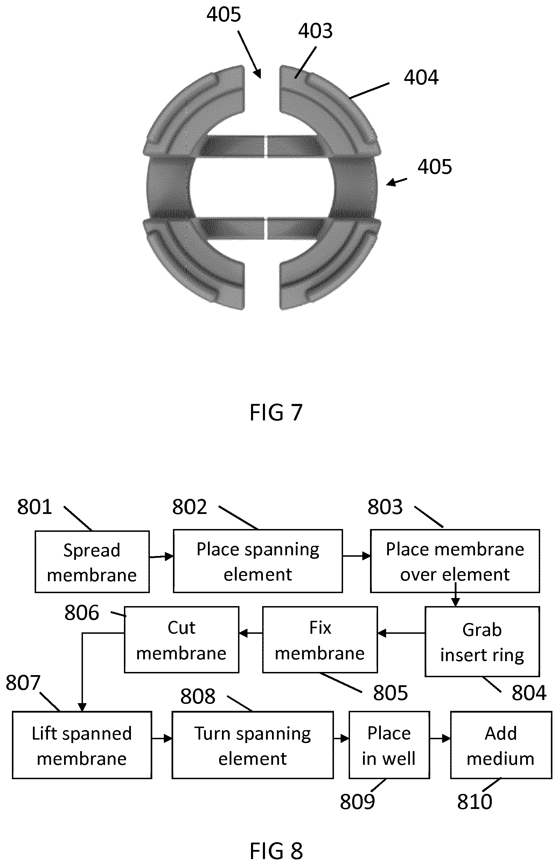

FIG 6 shows an exemplary handling means 401, including holding arms 402 which allow positioning the flexible element into the spanning element and to grab and lift the spanning device and the flexible element together and fixed to each other (and the spanned membrane held by both the device and the flexible element). For example the holding arms 402 may allow pressing the flexible element into the spanning element while a membrane is spanned over the spanning element, thereby fixing the membrane on the spanning element. The pressure may be done over portions of the perimeter of the inserting ring, for example by the lower surface 403 of the holding arms 402, which is clearly shown in the bottom view of FIG 7. The present invention may provide two, three or more contact points or areas at different positions along the perimeter of the flexible element for assisting in the positioning thereof over the spanning element.

The handling means 401 also may include at least one tab 404, which may help holding and positioning the flexible element 301. It may include also slots 405 for fitting the foot or feet 303 (either bent feet or straight feet) of the flexible element 302, if present.

The handling means 401 may function like a clamp, for example including handlers 406 and spring, or in general a fulcrum 407, between the handlers 406, which allows opening holding arms 402 by pressing on the handlers. This may release the flexible element 301 and/or the flexible element 301 and the spanning element.

The handling means may comprise resilient materials, which may be compatible with biological and/or chemical environments. For example, as before, it may comprise plastics (e.g. polypropylene).

The handling means typically may be construed to allow, during handling of the flexible ring, that the feet of the flexible ring, when present, fit in or be compatible with the handling means.

The membrane can be kept spanned and extended during manipulation, for example from the preparation environment towards a well plate. Chances of de-attachment of the membrane shaped tissue from the membrane spanning element are reduced. Additionally, the spanning device can be easily handled. While tweezers can still be used for handling a spanned membrane on the spanning element, for example by grasping a foot of the flexible element, higher control is provided by the handling means, which also allows positioning safely the flexible element. Using tweezers to position the flexible element requires much more skill and it is more prone to mistakes and damage on the membrane.

The handling tool may allow easy inspection of the membrane shaped tissue, as well as of cell cultures installed on the membrane shaped tissue, for example while still clamped by the handling means, before transferral to a well.

In some embodiments, the handling tool 100 includes materials that make it suitable for using it in an autoclave, so parts or the whole handling tool 100 is autoclavable.

In a second aspect, a method of providing a spanned membrane is provided. The method comprises placing the spanning device on a suitable protrusion of the platform, and placing the membrane over the spanning element and the upstanding protrusion. Then the flexible element is provided to the spanning device, fixing the membrane. This can be done with the positioning device. Then the membrane can be separated from the platform, while being held by the spanning device and the flexible element, by lifting them. This can be done again with the positioning device.

FIG 8 shows an exemplary workflow of such method. In a first step, the membrane is provided 801 on the platform and spread out, e.g. using tweezers. The researcher can now estimate the size of the spanning element that will be needed to fixate the membrane.

Next the spanning element is placed 802 in the designated upstanding protrusion of the platform (one that fits the spanning element, on choice of the user, e.g. according to the size of membranes). For example, it may be introduced in a slot provided by the space between the edge of a hole in the cutting support and the protrusion, or by the orifice 114 on the base plate 110 and the protrusion 111 (FIG 2).

The membrane is placed 803, e.g. dragged, over the spanning element. This can be done without completely lifting the membrane. This way the membrane will not completely stick to itself. When the membrane is placed over the spanning element, it can still be adjusted so that the ring can be placed on the edge of the membrane.

The top part of the protrusion may be adapted to hold the membrane without unnecessary tension or bending (e.g. by slightly protruding over the spanning device, or by being flush with the spanning device).

Then, the flexible element can be grabbed 804 and lifted using the handling means. The user does not need to pinch the handling means but can just press downward over the ring, thus slipping the (e.g. flexible) flexible element handling means. The handling means can be designed in such way that it clamps the flexible element automatically. For example, the tabs 404 (FIG 7) may provide extra clamping.

Then the membrane can be fixed 805. The handling means with the flexible element can be pressed down on top of the membrane. The flexible element is inserted on the spanning element, fixing the membrane. For example, the flexible ring 301 slides over the membrane and locks in the groove 202 of the hard ring.

Once fixed, the membrane can be cut 806, e.g. using a scalpel. It can also be done by punching. The flexible element is still locked in the groove of the spanning element, so that the membrane only has to be held from one side. This can be done by using tweezers. With the other free hand the user can cut the membrane as close to the edge of the flexible element as possible, thus providing reduction of trim loss.

Then the spanning element with the spanned membrane and the flexible element are lifted 807. The handling means can be used again to lift the spanning element and separate it from the protrusion of the platform. If the user chose the flexible ring with small feet, the insert can be lifted using tweezers also.

In some embodiments, the spanning element can be turned 808 upside down before placing 809 the membrane in a well of a wellplate. This can be done by, e.g., tweezers.

Inserting the spanning device with the spanned membrane shaped tissue into e.g. a well plate, where typically research and/or further actions are performed, can be easily done (e.g. by using the handling means, and/or tweezers). A flexible element with feet provided may also improve handling, and improve protection of the membrane (by reducing contact with support surfaces, for example).

Afterwards, normal process may follow, for example adding 810 medium to a fixated membrane.

The present method allows also using leftover membrane on the platform to fixate it in other spanning elements, for example with different sizes, and repeat the previous processes.

The present invention allows providing a system which can use different medium configurations. For example the system is allowed to work with a configuration wherein only a medium is present at the bottom side of the membrane, a configuration wherein different media is provided at both sides of the membrane or a configuration wherein the same media is provided at both sides of the membrane. The system thus provides flexibility on the amount and the position of where the medium is provided.

The system allows working with wet as well as with dry membranes. If working with wet membranes, drying of the medium can be reduced by e.g. the presence of the platform edges.

The spanning element of the present invention may fit most of the membranes, for example membrane shaped tissue with thickness between 20µm and 200µm.

A highly compact device, with small amount of parts, can be provided. It can be used within a flow hood, thus reducing hazard from fumes and the like, as the required movements of operation (e.g. from an operator or researched) are small movements.

Additionally, the flat platform and the addition of medium allows to easily spread the membrane. This gives the operator a complete overview of the surface. It improves area utilization and examination of the membrane.

The flat platform and the addition of medium also allow to easily slide, turn and position the membrane in all directions. The wide surface and/or material of the platform (e.g. of the cutting support, or optionally of the base plate) may allow these manipulations.

This allows the operator to position the membrane strategically and accurately over spanning elements, for example over spanning elements of different diameters.

This leads to reduction of trim loss.

In yet another aspect, the present invention also relates to the use of a tool according to an embodiment of the first aspect for spanning of an amniotic membrane.

## Claims

1. A tool for fixating and handling (100) membrane-shaped tissue for research or clinical use, the tool comprising
- a platform (101) comprising a base plate (110) having at least one positioning element (111) for positioning a spanning element on the platform,
- at least one round shaped spanning element (201) adapted for being positioned on the platform (101) using the positioning element (111),
- at least one flexible element (301) for positioning over the round shaped spanning element (201) for spanning the membrane shaped tissue, and
- at least one handling means (401) for handling the flexible element (301) during positioning of the flexible element (301) over the round shaped spanning element (201), and for handling the combination of the round shaped spanning element (201) and the flexible element (301), when the membrane is spanned over the round shaped spanning element, wherein the handling means (401) comprises holding arms having a round shaped end portion, the holding arms comprising tabs configured to provide the handling means (401) with at least two contact points or contact areas at different positions along the perimeter of the flexible element (301) for holding the flexible element (301-, when the flexible element (301) is positioned in the handling means (401).

2. The tool (100) according to claim 1, wherein the at least one round shaped spanning element (201) comprises a slot for locking the flexible element (301) in the slot when spanning the membrane over the round shaped spanning element (201).

3. The tool (100) according to any of the previous claims, wherein the positioning element (111) comprises upstanding features adapted for positioning the round shaped spanning element (201) over the upstanding features so as to fix the position of the round shaped spanning element (201) with respect to the base plate (110).

4. The tool (100) according to claim 3, wherein the upstanding features comprise at least one cylindrical shaped element, over which the round shaped spanning element (201) can be positioned.

5. The tool (100) according to claim 4, wherein the cylindrical shaped element is an upstanding protrusion extending from the bottom of the base plate (110).

6. The tool (100) according to any of the previous claims, furthermore comprising a cutting support (120) comprising one or more substantially round shaped holes (121), so that the round shaped spanning element (201) can be positioned on the base plate (110) in one of said substantially round shaped holes (121) of the cutting support (120) so that a top side of the round shaped spanning element (201), when positioned on the platform (101), is extending through or substantially flush with the surface of the cutting support (120), when the cutting support (120) is positioned on the base plate (110).

7. The tool (100) according to claim 6, wherein the cutting support (120) is made of a hard material with a good resistance to cutting with a scalpel, such as for example made of a metal, or wherein the cutting support is made of a soft material, such as self healing cutting mat PVC.

8. The tool (100) according to any of the previous claims, wherein the platform (101) comprises two or more positioning elements with different diameters for positioning round shaped spanning elements (201) with different diameter for preparing spanned membrane-shaped tissues with different diameter.

9. The tool (100) according to any of the previous claims, wherein the platform (101) has an upstanding edge to allow medium to be provided in the platform (101) for influencing the environment during spanning and prior to transferring the membrane e.g. to a research or processing tool.

10. The tool (100) according to any of the previous claims, wherein the different components of the tool (100) are provided in a sterilized way.

11. The tool (100) according to claim 10, wherein the different components are packaged together.

12. The tool (100) according to any of the previous claims, wherein the different components of the tool (100) are made of a material sterilizable at at least 80°C.

13. A method for fixating and handling membrane-shaped tissue for research or clinical use, the method comprising
- positioning at least one round shaped spanning element (201) on a platform (101) using a positioning element determining a position on the platform (101),
- spreading the membrane-shaped tissue over the round shaped spanning element (201),
- positioning at least one flexible element over the round shaped spanning element (201) for spanning the membrane shaped tissue using a handling means (401), wherein the handling means (401) comprises holding arms having a round shaped end portion, comprising tabs configured to provide the handling means (401) with at least two contact points or contact areas at different positions along the perimeter of the flexible element (301) for holding the flexible element (301), when the flexible element (301) is positioned in the handling means (401), and
- removing the combination of the round shaped spanning element (201) and the flexible element (301) when the membrane is spanned from the platform (101).

14. Use of a tool according to any of claims 1 to 12, for spanning of an amniotic membrane.

## Patentansprüche

1. Ein Werkzeug zum Fixieren und Handhaben (100) von membranförmigem Gewebe für Forschung oder klinische Nutzung, wobei das Werkzeug umfasst
- eine Plattform (101), die eine Grundplatte (110) umfasst, die mindestens ein Positionierungselement (111) zum Positionieren eines Spannelements auf der Plattform aufweist,
- mindestens ein rund geformtes Spannelement (201), das angepasst ist, um unter Verwendung des Positionierungselements (111) auf der Plattform (101) positioniert zu werden,
- mindestens ein flexibles Element (301) zur Positionierung über dem rund geformten Spannelement (201) zum Überspannen des membranförmigen Gewebes, und
- mindestens ein Handhabungsmittel (401) zum Handhaben des flexiblen Elements (301) während des Positionierens des flexiblen Elements (301) über dem rund geformten Spannelement (201) und zum Handhaben der Kombination aus dem rund geformten Spannelement (201) und dem flexiblen Element (301), wenn die Membran über das rund geformte Spannelement gespannt ist, wobei das Handhabungsmittel (401) Haltearme umfasst, die einen rund geformten Endabschnitt aufweisen, wobei die Haltearme Laschen umfassen, die konfiguriert sind, um dem Handhabungsmittel (401) mindestens zwei Kontaktpunkte oder Kontaktflächen an verschiedenen Positionen entlang des Umfangs des flexiblen Elements (301) zum Halten des flexiblen Elements (301) bereitzustellen, wenn das flexible Element (301) in dem Handhabungsmittel (401) positioniert ist.

2. Das Werkzeug (100) nach Anspruch 1, wobei das mindestens eine rund geformte Spannelement (201) einen Schlitz zum Verriegeln des flexiblen Elements (301) in dem Schlitz beim Spannen der Membran über das rund geformte Spannelement (201) umfasst.

3. Das Werkzeug (100) nach einem der vorstehenden Ansprüche, wobei das Positionierungselement (111) aufrecht stehende Merkmale umfasst, die zum Positionieren des rund geformten Spannelements (201) über den aufrecht stehenden Merkmalen angepasst sind, um die Position des rund geformten Spannelements (201) in Bezug auf die Grundplatte (110) zu fixieren.

4. Das Werkzeug (100) nach Anspruch 3, wobei die aufrecht stehenden Merkmale mindestens ein zylindrisch geformtes Element umfassen, über dem das rund geformte Spannelement (201) positioniert werden kann.

5. Das Werkzeug (100) nach Anspruch 4, wobei das zylindrisch geformte Element ein aufrecht stehender Vorsprung ist, der sich von der Unterseite der Grundplatte (110) erstreckt.

6. Das Werkzeug (100) nach einem der vorstehenden Ansprüche, das darüber hinaus eine Schneidauflage (120) umfasst, die eine oder mehrere im Wesentlichen rund geformte Öffnungen (121) umfasst, sodass das rund geformte Spannelement (201) auf der Grundplatte (110) in einem der im Wesentlichen rund geformten Löcher (121) der Schneidauflage (120) positioniert werden kann, sodass sich eine Oberseite des rund geformten Spannelements (201), wenn es auf der Plattform (101) positioniert ist, durch die Oberfläche der Schneidauflage (120) hindurch erstreckt oder im Wesentlichen damit bündig abschließt, wenn die Schneidauflage (120) auf der Grundplatte (110) positioniert ist.

7. Das Werkzeug (100) nach Anspruch 6, wobei die Schneidauflage (120) aus einem harten Material mit guter Schnittfestigkeit gegenüber einem Skalpell gefertigt ist, beispielsweise aus Metall gefertigt ist, oder wobei die Schneidauflage aus einem weichen Material, beispielsweise einer selbstheilenden Schneidmatte aus PVC gefertigt ist.

8. Das Werkzeug (100) nach einem der vorstehenden Ansprüche, wobei die Plattform (101) zwei oder mehr Positionierungselemente mit unterschiedlichen Durchmessern zum Positionieren von rund geformten Spannelementen (201) mit unterschiedlichem Durchmesser zur Herstellung von gespannten membranförmigen Geweben mit unterschiedlichem Durchmesser umfasst.

9. Das Werkzeug (100) nach einem der vorstehenden Ansprüche, wobei die Plattform (101) eine aufrecht stehende Kante aufweist, um zu ermöglichen, dass ein Medium in der Plattform (101) zum Beeinflussen der Umgebung beim Spannen und vor dem Übertragen der Membran, z. B. auf ein Forschungs- oder Verarbeitungswerkzeug bereitgestellt wird.

10. Das Werkzeug (100) nach einem der vorstehenden Ansprüche, wobei die verschiedenen Komponenten des Werkzeugs (100) auf sterilisierte Weise bereitgestellt werden.

11. Das Werkzeug (100) nach Anspruch 10, wobei die verschiedenen Komponenten zusammen verpackt sind.

12. Das Werkzeug (100) nach einem der vorstehenden Ansprüche, wobei die verschiedenen Komponenten des Werkzeugs (100) aus einem bei mindestens 80°C sterilisierbaren Material gefertigt sind.

13. Ein Verfahren zum Fixieren und Handhaben von membranförmigem Gewebe für Forschung oder klinische Nutzung, wobei das Verfahren umfasst
- Positionieren mindestens eines rund geformten Spannelements (201) auf einer Plattform (101) unter Verwendung eines Positionierungselements, das eine Position auf der Plattform (101) bestimmt.
- Ausbreiten des membranförmigen Gewebes über das rund geformte Spannelement (201),
- Positionieren mindestens eines flexiblen Elements über dem rund geformten Spannelement (201) zum Überspannen des membranförmigen Gewebes unter Verwendung eines Handhabungsmittels (401), wobei das Handhabungsmittel (401) Haltearme umfasst, die einen runden Endabschnitt aufweisen, Laschen umfassend, die konfiguriert sind, um dem Handhabungsmittel (401) mindestens zwei Kontaktpunkte oder Kontaktflächen an verschiedenen Positionen entlang des Umfangs des flexiblen Elements (301) zum Halten des flexiblen Elements (301) bereitzustellen, wenn das flexible Element (301) in dem Handhabungsmittel (401) positioniert ist, und
- Entfernen der Kombination aus dem rund geformten Spannelement (201) und dem flexiblen Element (301), wenn die Membran von der Plattform (101) überspannt wird.

14. Verwendung eines Werkzeugs nach einem der Ansprüche 1 bis 12 zum Überspannen einer Amnionmembran.

## Revendications

1. Un outil pour la fixation et la manipulation (100) de tissus en forme de membrane à des fins de recherche ou d'utilisation clinique, ledit outil comprenant
- une plateforme (101) comprenant une plaque de base (110) ayant au moins un élément de positionnement (111) pour positionner un élément d'étirement sur la plateforme,
- au moins un élément d'étirement de forme ronde (201) adapté pour être positionné sur la plateforme (101) en utilisant l'élément de positionnement (111),
- au moins un élément flexible (301) pour être positionné sur l'élément d'étirement de forme ronde (201) pour étirer le tissu en forme de membrane, et
- au moins un moyen de manipulation (401) pour manipuler l'élément flexible (301) lors du positionnement de l'élément flexible (301) sur l'élément d'étirement de forme ronde (201), et pour manipuler la combinaison de l'élément d'étirement de forme ronde (201) et de l'élément flexible (301), lorsque la membrane est étirée sur l'élément d'étirement de forme ronde, dans lequel le moyen de manipulation (401) comprend des bras de maintien ayant une extrémité de forme ronde, lesdits bras de maintien comprenant des languettes configurées pour fournir au moyen de manipulation (401) au moins deux points de contact ou zones de contact à différentes positions le long du périmètre de l'élément flexible (301) pour maintenir l'élément flexible (301), lorsque l'élément flexible (301) est positionné dans le moyen de manipulation (401).

2. L'outil (100) selon la revendication 1, dans lequel ledit au moins un élément d'étirement de forme ronde (201) comprend une fente pour verrouiller l'élément flexible (301) dans ladite fente lors de l'étirement de la membrane sur l'élément d'étirement de forme ronde (201).

3. L'outil (100) selon l'une quelconque des revendications précédentes, dans lequel l'élément de positionnement (111) comprend des caractéristiques en saillie adaptées pour positionner l'élément d'étirement de forme ronde (201) sur lesdites caractéristiques en saillie de manière à fixer la position de l'élément d'étirement de forme ronde (201) par rapport à la plaque de base (110).

4. L'outil (100) selon la revendication 3, dans lequel lesdites caractéristiques en saillie comprennent au moins un élément de forme cylindrique, sur lequel l'élément d'étirement de forme ronde (201) peut être positionné.

5. L'outil (100) selon la revendication 4, dans lequel ledit élément de forme cylindrique est une saillie en relief s'étendant depuis le fond de la plaque de base (110).

6. L'outil (100) selon l'une quelconque des revendications précédentes, comprenant en outre un support de découpe (120) comprenant un ou plusieurs trous de forme sensiblement ronde (121), de sorte que l'élément d'étirement de forme ronde (201) puisse être positionné sur la plaque de base (110) dans l'un desdits trous de forme sensiblement ronde (121) du support de découpe (120) de sorte qu'un côté supérieur de l'élément d'étirement de forme ronde (201), lorsqu'il est positionné sur la plateforme (101), s'étend à travers ou est sensiblement affleurant avec la surface du support de découpe (120), lorsque le support de découpe (120) est positionné sur la plaque de base (110).

7. L'outil (100) selon la revendication 6, dans lequel ledit support de découpe (120) est fabriqué d'un matériau dur avec une bonne résistance à la découpe avec un scalpel, tel que par exemple fabriqué en métal, ou dans lequel le support de découpe est fabriqué d'un matériau souple, tel qu'un tapis de découpe auto-cicatrisant en PVC.

8. L'outil (100) selon l'une quelconque des revendications précédentes, dans lequel la plateforme (101) comprend deux ou plusieurs éléments de positionnement avec des diamètres différents pour positionner des éléments d'étirement de forme ronde (201) avec des diamètres différents pour préparer des tissus en forme de membrane étirés avec des diamètres différents.

9. L'outil (100) selon l'une quelconque des revendications précédentes, dans lequel la plateforme (101) a un bord en saillie pour permettre à un milieu d'être fourni dans la plateforme (101) pour influencer l'environnement pendant l'étirement et avant de transférer la membrane, par exemple, à un outil de recherche ou de traitement.

10. L'outil (100) selon l'une quelconque des revendications précédentes, dans lequel les différents composants de l'outil (100) sont fournis de manière stérilisée.

11. L'outil (100) selon la revendication 10, dans lequel lesdits différents composants sont emballés ensemble.

12. L'outil (100) selon l'une quelconque des revendications précédentes, dans lequel les différents composants de l'outil (100) sont fabriqués d'un matériau stérilisable à au moins 80°C.

13. Un procédé pour la fixation et la manipulation de tissus en forme de membrane à des fins de recherche ou d'utilisation clinique, ledit procédé comprenant
- le positionnement d'au moins un élément d'étirement de forme ronde (201) sur une plateforme (101) en utilisant un élément de positionnement déterminant une position sur la plateforme (101),
- l'étalement du tissu en forme de membrane sur l'élément d'étirement de forme ronde (201),
- le positionnement d'au moins un élément flexible sur l'élément d'étirement de forme ronde (201) pour étirer le tissu en forme de membrane en utilisant un moyen de manipulation (401), dans lequel ledit moyen de manipulation (401) comprend des bras de maintien ayant une extrémité de forme ronde, comprenant des languettes configurées pour fournir au moyen de manipulation (401) au moins deux points de contact ou zones de contact à différentes positions le long du périmètre de l'élément flexible (301) pour maintenir l'élément flexible (301), lorsque l'élément flexible (301) est positionné dans le moyen de manipulation (401), et
- le retrait de la combinaison de l'élément d'étirement de forme ronde (201) et de l'élément flexible (301) lorsque la membrane est étirée depuis la plateforme (101).

14. Utilisation d'un outil selon l'une quelconque des revendications 1 à 12, pour l'étirement d'une membrane amniotique.
